# EUROPEAN PATENT SPECIFICATION

(11) **EP 0 373 218 B1**
(45) Date of publication and mention of the grant of the patent: **26.05.1993**
(21) Application number: 88903384.1
(22) Date of filing: 19.04.1988
(51) Int. Cl.: A61K 37/32

(54) **KIDNEY GROWTH ACCELERATOR AND PROCESS FOR ITS PREPARATION**
NIEREN-WACHSTUMSBESCHLEUNIGER UND VERFAHREN ZU SEINER HERSTELLUNG
ACCELERATEUR DE LA CROISSANCE RENALE ET PROCEDE DE PREPARATION DUDIT ACCELERATEUR

(30) Priority: 20.04.1987 JP 95277/87
(43) Date of publication of application: 20.06.1990
(73) Proprietor: The Calpis Food Industry Co., Ltd., Shibuya-ku Tokyo (JP)
(72) Inventor: NOMURA, Kaoru, Musasino-shi Tokyo 180 (JP); OHMURA, K., The Calpis Food Industry Co.Ltd., Shibuya-ku, Tokyo 150 (JP); SHIRAKURA, Yuri, The Calpis Food Industry Co.Ltd., Shibuya-ku, Tokyo 150 (JP); NAKAMURA, Y., The Calpis Food Industry Co.Ltd., Shibuya-ku, Tokyo 150 (JP)
(74) Representative: Thomsen, Dieter, Dr.
(86) International application number: JP8800385
(87) International publication number: WO8808307

(56) References cited:
- FR-A- 2 489 151
- CHEMICAL ABSTRACTS, vol. 102, no.11, 18 March 1985, Columbus, OH (US); no. 90348w#
- ENDOCRINOLOGY, vol. 116, no. 2; K. NOMURA et al., pp. 616-621#

## Description

The invention relates to the use of luteinizing hormone or an isoform or mixture or isoforms of luteinizing hormone for manufacture of a medicament for promoting renal growth in humans.

From CA 102, No. 90 348 W (1985) and "Endocrinology" 116, pages 616 ff a study of Nomura et al. is known involving the observation of renal cells obtained from rats which have been treated with LH-RH. In detail, kidney slices were obtained from castrated-hypophysectomized male rats treated with a single injection of several different gonadotropin preparations (two ovine LH fractions, one bovine LH fraction, and one hCG fraction) or vehicle, then incubated in a buffer containing [³H]thymidine. Only one of the above, an ovine LH preparation, increased [³H]thymidine incorporation into renal DNA, with a peak occurring 8-10 h after injection and therefore termed renotropin. However, in hypophysectomized rats with intact testes, such an effect was not observed. Furthermore, while testosterone propionate alone did not alter basal incorporation in castrated-hypophysectomized rats, it abolished the incorporation that was stimulated by renotropin. These results suggest that androgens, whether of testicular origin or exogenously administered, suppress the increased incorporation of [³H]thymidine stimulated by renotropin. This antagonistic effect of androgen was also observed with T₄, but to a lesser degree. The findings confirm the presence of renotropin, which could not be attributed to other known pituitary hormones, and suggest that there is a complex interaction between this factor and two other renal growth-promoting hormones, testosterone and T₄.

Nothing is said in this prior art about the promotion of renal growth in humans, i. e. a particularly important medicinal field, as is generally known. Thus the object of the present invention is directed to improvements in this medicinal field in order to create medical means suitable for humans in the treatment of kidneys.

According to the invention this object is solved by the above mentioned use of the claim , i. e. for the manufacture of a corresponding medicament.

The production of an isoform or mixture of isoforms of luteinizing hormone having a renal growth promoting activity comprises fractionating and purifying luteinizing hormone according to the difference in isoelectric point.

In more detail it can be seen that the use of luteinizing hormone or an isoform or mixture of isoforms of luteinizing hormone for promoting renal growth in humans has now become applicable.

In "Biology of Reproduction" 34, 571-578 (1986) the existence of different forms of some hormones has been described, and the designation "isoforms" has been used in this context. Thus the terms "isomer" and "isomers" as far as used hereinafter mean "isoform" and "isoforms", respectively.

In the present case a renal growth promoter is concerned which comprises an isomer of luteinizing hormone having an activity of promoting renal growth as an active ingredient. It has been found that the administration of the renal growth promoter increases the number of renal cells or enhances the renal function; and thus the kidney is activated when it is suffering from a decrease of renal cells or a lowering in the renal function.

As a background within these investigations it has been considered that hypophysectomized mice would show a significant decrease in the renal weight, and furthermore that there might be some factor controlling renal growth in the pituitary. Thus it was attempted to find out a renal growth factor present in pituitary. Consequently it was isolated a hormone-like component promoting renal growth from the pituitary; and it was named RGF (or renotropin) (cf. Nippon Rinsho, 44 (1), separate issue, 84 - 88 (Jan. 1986)).

However it has been found that the RGF thus obtained is a crude product which shows an unstable activity and contains various contaminants having antagonistic effects.

In order to obtain the RGF as a single component the following way has been taken, i. e. a single band of an active ingredient can be obtained by fractionating and purifying the crude RGF isolated above according to the difference in isoelectric point.

The analysis of this single band and the determination of the primary structure thereof have surprisingly revealed that it is an isomer of luteinizing hormone.

It is known that luteinizing hormone is not a single compound, but a glycoprotein comprising a plurality of isomers. This is caused by the microstructural polymorphism thereof.

It was reported that ovine luteinizing hormone had heterogeneous amino-terminal sequences on the α subunit and heterogeneous carboxyl-terminal sequences on the β subunit (D.N. Ward, Int. J. Peptide Protein Res., 27, 70 - 78 (1986)). It was further reported that human luteinizing hormone was divided into seven peaks by column electrofocusing (cf. M. Yano, J. Jap. Tocol. Gynecol., 37 (5), 703 - 712 (1985)).

Thus, it has never been observed that luteinizing hormone and isomers thereof would have an effect of promoting renal growth.

It has been shown by practising of the present invention that the highest activity of promoting renal growth is achieved by an isomer of luteinizing hormone wherein a subunit α₃ is bound to another subunit β₃; and thus the renal growth promoter comprises a corresponding isomer of luteinizing hormone as an active ingredient. It is preferable that this isomer of luteinizing hormone comprises a subunit α₃ bound to another subunit β₃.

The process for the production of the isomer of luteinizing hormone having an activity of promoting renal growth compprises fractionating and purifying a crude renal growth factor according to the difference in isoelectric point.

The fractionation and purification treatment according to the difference in isoelectric point as described herein means a treatment whereby ampholytes such as proteins are fractionated from each other according to the inherent isoelectric point of each material and then separately collected. It may be carried out by, for example, chromatofocusing or electrofocusing.

As the crude renal growth factor to be used as the starting material in the present invention, crude luteinizing hormone extracts obtained from urine, blood or organs of mammals such as mouse, sheep or swine may be employed. Alternatively a crude product obtained from a culture system which is artificially prepared through genetic engineering may be used. Further a material obtained from a synthetic chemical reaction system may be employed. Anyway, a mixture of an isomer of luteinizing hormone having an effect of promoting renal growth with those having no such an effect is employed as the starting material.

For example, the crude renal growth factor is obtained from an organ in the following manner. An aqueous soltuion of ammonium sulfate is added to the pituitary and the resulting mixture is homogenized and centrifuged. The supernatant is purified and lyophilized. Then the lyophilized material is dissolved and purified by a series of chromatography treatments, for example with types of Sephadex (reg. TM) CM cellulose, Con-A etc. The result is a crude renal growth factor (RGF).

The crude renal growth factor (RGF) thus obtained is fractionated and purified according to the difference in isoelectric point to thereby give a material having an activity of promoting renal growth as a single band.

The material having an activity of promoting renal growth thus obtained as a single band may be separated into constituting subunits α and β. As a matter of course, the renal growth promoting activity would disappear after dividing the material into these subunits.

The separation may be carried out by, for example, reverse phase liquid chromatography. In the separation pattern, the subunits α and β show each a plurality of peaks depending on the chain length of amino acids. It has been confirmed that these peaks correspond to the several shorter chains of the amino acids obtained by cutting the subunits α and β, which chains had been reported previously.

Although a material wherein a subunit α₃ is bound to another subunit β₃ has a slight luteinizing hormone activity, it is different from other isomers of luteinizing hormone in that it has a remarkable renal growth promoting activity.

The subunits α₃ and β₃ are those which suffer from the least cuttings. Among various combinations of the subunits which are bound to each other again, the one having the subunits α₃ and β₃ showed the highest activity of promoting renal growth.

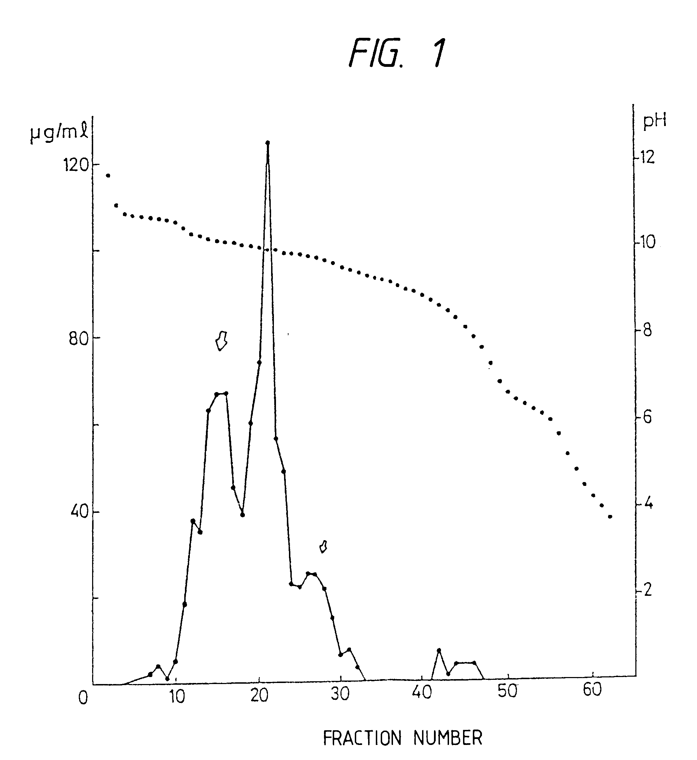

In the drawing Fig. 1 shows the protein content of each fraction obtained by isoelectric focusing of a crude renal growth factor in Example 1.

The following examples describe the invention in more details.

### Example 1

10 mM of phenylmethylsulfonyl fluoride was added to approximately 350 g of porcine pituitary and 1 ℓ of a 0.15 M aqueous solution of ammonium sulfate. The resulting mixture was homogenized in a Waring blender for two minutes while cooling. After adjusting the pH value to 4.0, the homogenized mixture was allowed to stand for a sufficient period of time under cooling. Then it was centrifuged and the supernatant was coolected. After further adjusting the pH value to 3.0 with a 0.5 M aqueous solution of metaphosphoric acid, the resulting solution was centrifuged and the supernatant was collected. After adjusting the pH value of the supernatant to 6.5 to 7.0, ammonium sulfate was added thereto until 50 % saturation was achieved. Then the mixture was allowed to stand for a sufficient period of time again under cooling. Subsequently it was centrifuged. The precipitate was collected and suspended in 50 to 100 ml of a 0.2 M solution of dibasic potassium phosphate. The suspension was transferred into a dialysis tube and sufficiently dialyzed under cooling. Then the content was taken out and the pH value thereof was again adjusted to 8.5. The obtained material was lyophilized.

The lyophilized powder thus obtained was dissolved in a 0.01 M solution of dibasic sodium phosphate and applied to a SP-Sephadex® column equilibrated with the a.m. buffer. Fractions eluated with 0.1 M dibasic sodium phosphate were collected and lyophilized.

The lyophilized powder was dissolved in a small amount of a 0.05 M solution of ammonium hydrogencarbonate and fractionated through a molecular sieve by using a Sephadex® G-100 column wherein the same buffer system was employed. Fractions of molecular weights of approximately 40,000 were collected and lyophilized.

The dry powder thus obtained was dissolved in a small amount of a solution of 10 mM Tris-HCl (pH 7.5) and 0.3 M sodium chloride and then fractionated by using a Concanavalin A-Sepharose (reg. TM) column wherein the same buffer system was employed. Fractions eluated with 0.2 M methyl mannoside were collected, sufficiently dialyzed under cooling and then lyophilized. Thus approximately 25 mg of a crude renal growth factor (RGF) was obtained.

Subsequently this crude product was fractionated and purified according to the difference in isoelectric point by using an LKB isoelectric focusing column of 100 ml capacity product of (LKB Produkter AB). As carrier ampholytes, Ampholine pH 3.5 - 10, Ampholine pH 9 - 11 and Ampholine pH 7 - 9 were employed, (Ampholine = reg. TM). A sorbitol density gradient (5 to 50 %) was prepared and the a.m. crude product dissolved in 2 ml of a dense solution was placed onto the center of the column (dense gradient solution: sorbitol 27 g, distilled water 34.9 ml, Ampholine (pH 9-11) 2.1 ml; light gradient solution: sorbitol 2.7 g, distilled water 52.3 ml, Ampholine (pH 9-11) 0.3 ml, Ampholine (pH 3.5-10) 0.2 ml, Ampholine (pH 7-9) 0.2 ml). A 1 M sodium hydroxide solution and a 0.01 M acetic acid solution were used as electrode solutions. Electricity at 800-1500 V was turned on at 4°C for 24 hours. After the completion of the turning of electricity, 1.5-ml portions of the material were collected with a fraction collecter. Then the pH value of each fraction at 4°C was immediately determined (COM-11: product of Denki Kagaku Kogyo K.K., electrode type CE 105 C, standard buffer solution: product of Wako Pure Chemicals Co., Inc.). Simultaneously the protein content of each fraction was determined. Fig. 1 shows the chromatographed pattern.

As a result, 104 µg, 281 µg, 415 µg and 103 µg of fractions of a pI higher than 10.32, that of 10.32 to 10.15, that of 10.15 to 9.89 and that lower than 9.89 were obtained respectively from approximately 1.5 mg of the crude renal growth factor.

Each fraction was sufficiently dialyzed under cooling to in order remove Ampholines and sorbitol, and then lyophilized to give a powder.

The crude renal growth factor was purified several times by repeating the isoelectric focusing under the same conditions as those employed above. Then fractions of the same pI range were combined together. The pI of equine cardiac myoglobin (Type III: product of Sigma Chemical Co.) used as a standard protein was 7.97.

### Evaluation of renal growth promoting activity:

It was determined which fraction among those obtained above would promote renal growth.

The renal growth promoting effect of renotropin was determined with the guidance of the promotion of the DNA-synthesizing effect of renal cells.

Male CD rats (born on the same day; average body weight: 100 g) were subjected to hypophysectomy and castration and then fed for nine days. After confirming whether the operation had been appropriately carried out or not, the rats were divided into test lots and a control lot each consisting of at least five animals.

50 µg of each fraction fractionated by the above isoelectric focusing was dissolved in 150 µl of a 50 mM borate buffer (pH 7.5) containing 0.1 % of BSA and subcutaneous injected into each rat. After eight hours, the animal was sacrificed by cutting off the head and blood-drawn, and then the right and left kidneys were taken out. After peeling off the capsule, two slices of each kidney were prepared along the corticomedullary axis. These slices were incubated in 2 ml of Krebs-Ringer bicarbonate buffer (pH 7.5) at 37°C for two hours. This buffer had been preliminarily aerated with a gas mixture comprising 95 % of oxygen and 5 % of carbon dioxide and then 74 kBg/ml (2 µCi/ml) of 1,2-methyl³H-thymidine product of New England Nuclear Corp.) had been added thereto; the unit 1 µCi is= 3,7 x 10⁻⁴ S⁻¹. After the completion of the incubation, the slices were rinsed with a 4 mM thymidine solution and stored at - 20°C.

4 ml of distilled water was added to the stored slices and the resulting mixture was homogenized with the use of an ultradisperser product of Yamato Kagaku K.K.). Then DNA contained therein was extracted according to a process proposed by A. Fleck and H. M. Munro (cf. Biochim. Biophys. Acta, 55, 571 (1962)). The extracted DNA was hydrolyzed according to a process reported by J.R.W. Wannemacher, J.W.L. Banks and W.H. Wunner (Anal. Biochem., 11, 320 (1965)) and then determined by using bovine thymus DNA (type I, product of Sigma Chemical Co.) as a standard DNA. Simultaneously the radioactivity of 0.5 ml of the extracted DNA was determined in a conventional manner.

The DNA synthesis promoting activity of the renal cells was determined by radioactivity/total DNA content and expressed in the percentage of each lot by defining that of the control lot as 100 %. As a result, the fraction of a pI higher than 10.32 showed a DNA synthesis promoting activity of 109 %, the fraction of a pI of 10.32 to 10.15 showed that of 130 % (thick arrow in Fig. 1), the fraction of a pI of 10.15 to 9.89, which showed the largest protein content, showed no DNA synthesis promoting effect (98%) and the fraction of a pI lower than 9.89 showed that of 115% (thin arrow in Fig. 1). Namely, the fraction of a pI of 10.32 to 10.15 and that of a pI lower than 9.89 (9.89 to 9.32) contained a renal growth promoting component. In addition, the fraction of a pI higher than 10.32 also contained the renal growth promoting component, though the activity thereof was slight.

The DNA synthesis promoting effect of the crude renal growth factor of the same batch was determined at the same time. Thus 112 % of the activity was observed.

### Effect of renal growth promoting component on renal growth in vivo:

Male CD-1 mice (born on the same day; average body weight: 17 g) were subjected to hypophysectomy and castration and then fed for nine days. After confirming whether the operation had been appropriately carried out or not, the mice were divided into a test lot and a control lot each consisting of ten animals. 40 µg of the fraction of a pI of 10.32 to 10.15 as obtained above by isoelectric focusing was dissolved in 150 µl of 50 mµl borate buffer (pH 7.5) containing a 0.1 % BSA solution. The other 40 µg portions of the fraction of a pI of 10.32 to 10.15 were treated in the same manner. The resulting solution was subcutaneously injected into each animal at a dose of 150 µl/day continuously for five days. On the sixth day, the animal was sacrificed by cutting off the head and blood-drawn, and then the right and left kidneys were taken out. After peeling off the capsule, these kidneys were lyophilized. The ratio of the weight of the dry kidneys to the final body weight of the rat was calculated and the difference in the kidney weight between the test and control lots was examined. As a result, the test lot showed an increase in the kidney weight of 110 %, which suggests that the employed fraction had a renal growth promoting effect.

### Separation and rebinding of subunits α and β in renal growth promoting component:

The fraction of a pI of 10.32 to 10.15 fractionated by isoelectric focusing was subjected to high-performance liquid chromatography and subunits thus separated were collected. The collected matter was dissolved in a small amount of a 0.1 % aqueous solution of trifluoroacetic acid (TFA) and then separated with a reversed phase column (Baker Bond wide pore butyl C₄: product of Baker Research Products) at a linear gradient (10% - 50%) with the use of 2-propanol/acetonitrile (7 : 3) containing 0.1 % of TFA as a solvent.

Thus the subunits α and β were obtained each as three main peaks named α₁, α₂ and α₃; and β₁, β₂ and β₃ in the order of elution. When 3 mg of the sample was separated, 230 µg of α₁, 450 µg of α₂, 350 µg of α₃, 410 µg of β₁, 570 µg of β₂ and 60 µg of β₃ were obtained. As the result of the analysis of amino acid composition, etc., it was found that α₃ and β₃ were luteinizing hormone isomers decomposed to the lowest extent.

Equimolar amounts of α₁ and β₁ ; α₂ and β₂; and α₃ and β₃ were mixed together. Each mixture thus obtained was maintained at 37°C in a small amount of a 1 % aqueous solution of ammonium carbonate to thereby attempt to associate the subunit α with the subunit β. Subsequently the mixture was desalted with a TSK gel G 3000 SW column (product of Toyo Soda Mfg. Co., Ltd.) and then lyophilized. The activities of these samples were compared with one another based on the DNA synthesis promoting effects as described above. As a result, the combination of α₃/β₃ showed the highest activity, i.e., approximately 2.5 times as high as that of the combination of α₁/β₁ in the difference from the control lot.

### Example 2

100 g of an ovine pituitary powder (product of Waitaki NZ Refrigerating Ltd.) was suspended in 400 ml of cold water and then 1.5 ℓ of a 0.15 M ammonium sulfate solution was added thereto. 10 mM of phenylmethylsulfonyl fluoride was added thereto and the resulting mixture was homogenized with Polytron under cooling. After adjusting the pH value to 4.0, the homogenized mixture was allowed to stand for a sufficient period of time under cooling and then centrifuged. The supernatant was collected and treated in the same manner as described in Example 1. Then it was treated with an SP-Sephadex® column, a Sephadex® G-100 column and a Concanavalin A-Sepharose® column to thereby give a crude renal growth factor.

Further 5 mg of the crude renal growth factor was fractionated with isoelectric focusing, as in Example 1. As a result, a fraction of a pI higher than 9.85, that of a pI of 9.85 to 9.60, that of a pI of 9.60 to 9.10, that of a pI of 9.10 to 8.60, that of a pI of 8.60 to 7.30 and that of a pI lower than 7.30 were obtained. As the result of the same bioassay as described in Example 1, it was confirmed that the fraction of a pI higher than 9.85 contained a renal growth promoting component.

### Example 3

750 ml of an extract of human pituitary product of KabiVitrum AB) was centrifuged. The resulting precipitate was collected and suspended in 70 ml of a 0.2 M aqueous solution of dibasic potassium phosphate. The resulting suspension was heated to 60°C for three minutes and then dialyzed for two days under cooling. The content was taken out and 0.13 M ammonium sulfate was added thereto. After adjusting the pH value to 4.0, the pH value of the mixture was adjusted again to 3.0 with a 0.5 M metaphosphoric acid solution. Then the mixture was allowed to stand under cooling and centrifuged. The supernatant was collected, sufficiently dialyzed under cooling and then lyophilized.

Then it was treated in the same manner as described in Example 1 with the use of an SP-Sephadex® column and a Sephadex G-100® column to thereby give an extract of crude luteinizing hormone.

This crude luteinizing hormone extract was treated with a Concanavalin A-Sephadex column to thereby give a crude renal growth factor.

The obtained crude renal growth factor was fractionated by isoelectric focusing. Each fraction thus obtained was subjected to the same bioassay as described in Example 1. As a result, it was confirmed that a fraction of a pI of 9.05 to 9.90 contained a renal growth promoting component.

## Claims

1. Use of luteinizing hormone or an isoform or mixture of isoforms of luteinizing hormone for manufacture of a medicament for promoting renal growth in humans.

## Patentansprüche

1. Verwendung des luteinisierenden Hormons oder eines Isoformen oder Gemisches von Isoformen des luteinisierenden Hormons zur Herstellung eines Medikaments zur Förderung des renalen Wachstums bei Menschen.

## Revendications

1. Utilisation de l'hormone lutéinisante ou d'une isoforme ou d'un mélange d'isoformes de l'hormone lutéinisante pour la fabrication d'un médicament destiné à promouvoir la croissance rénale chez les êtres humains.
